# EUROPÄISCHE PATENTSCHRIFT

(11) **EP 1 352 050 B1**
(45) Veröffentlichungstag und Bekanntmachung des Hinweises auf die Patenterteilung: **17.12.2008**
(21) Anmeldenummer: 02709997.7
(22) Anmeldetag: 08.01.2002
(51) Int. Cl.: C12N 1/20, C12N 9/78, C12P 13/02

(54) **MIKROBIOLOGISCHES VERFAHREN ZUR HERSTELLUNG VON AMIDEN**
MICROBIOLOGICAL METHOD FOR PRODUCING AMIDES
PROCEDE MICROBIOLOGIQUE POUR LA PRODUCTION D'AMIDES

(30) Priorität: 09.01.2001 EP 01100493; 27.12.2001 US 342373 P
(43) Veröffentlichungstag der Anmeldung: 15.10.2003
(73) Patentinhaber: Lonza AG, 4052 Basel (CH)
(72) Erfinder: ROBINS, Karen, Tracey, CH-3930 Visp (CH); NAGASAWA, Toru, Gifu 501-11 (JP)
(74) Vertreter: Riegler, Norbert Hermann
(86) Internationale Anmeldenummer: PCT/EP2002/000103
(87) Internationale Veröffentlichungsnummer: WO 2002/055670

(56) Entgegenhaltungen:
- EP-A- 0 307 926
- DE-C- 4 313 649
- LANGDAHL BJARNE R ET AL: "Nitrile hydrolysis of Rhodococcus erythropolis BL1, an acetonitrile-tolerant strain isolated from a marine sediment." MICROBIOLOGY (READING), Bd. 142, Nr. 1, 1996, Seiten 145-154, XP001037265 ISSN: 1350-0872
- TORU NAGASAWA ET AL: "NITIRLE HYDRATASE-CATALYZED PRODUCTION OF NICOTINAMIDE FROM 3-CYANOPYRIDINE IN RHODOCOCCUS THODOCHROUS J1" APPLIED AND ENVIRONMENTAL MICROBIOLOGY, WASHINGTON,DC, US, Bd. 54, Nr. 7, 1. Juli 1988 (1988-07-01), Seiten 1766-1769, XP000106560 ISSN: 0099-2240
- WATANABE I ET AL: "SCREENING ISOLATION AND TAXONOMICAL PROPERTIES OF MICROORGANISMS HAVING ACRYLONITRILE-HYDRATING ACTIVITY" AGRICULTURAL AND BIOLOGICAL CHEMISTRY, Bd. 51, Nr. 12, 1987, Seiten 3193-3200, XP001062886 ISSN: 0002-1369
- ASANO Y ET AL: "ALIPHATIC NITRILE HYDRATASE FROM ARTHROBACTER SP. J-1 PURIFICATION AND CHARACTERIZATION" AGRICULTURAL AND BIOLOGICAL CHEMISTRY, JAPAN SOC. FOR BIOSCIENCE, BIOTECHNOLOGY AND AGROCHEM. TOKYO, JP, Bd. 5, Nr. 46, Mai 1982 (1982-05), Seiten 1165-1174, XP001068503 ISSN: 0002-1369

## Beschreibung

Die Erfindung betrifft Mikroorganismen, die befähigt sind, Acetonitril in Konzentrationen von mindestens 3 M zu tolerieren, ein Enzym mit Nitrilhydratase-Aktivität, ein Verfahren zur Herstellung von Amiden unter Verwendung dieser Mikroorganismen bzw. des Enzyms, sowie die Verwendung dieser Mikroorganismen zur Beseitigung von Acetonitril-Abfällen.

Für die Herstellung von Amiden wie beispielsweise Nikotinsäureamid, einem für Tier und Mensch essentiellen Vitamin des Vitamin-B-Komplexes, sind bereits mehrere biotechnologische Verfahren bekannt.

Beispielsweise beschreibt EP-A 0 307 926 die Umsetzung von 3-Cyanpyridin zu Nikotinsäureamid mittels *Rhodococcus rhodochrous* J1. Dieses Verfahren hat den Nachteil, dass *Rhodococcus rhodochrous* J1 rot gefärbt ist und demzufolge eine Verfärbung des Produktes stattfindet. Des weiteren weist dieser Mikroorganismus einen hohen K_{M}-Wert bezüglich des Substrates 3-Cyanpyridin auf, hat eine geringe Temperaturtoleranz und eine geringe Toleranz bezüglich 3-Cyanypyridin.

WO 99/05306 beschreibt beispielsweise ein Verfahren zur Herstellung von Nikotinsäureamid ausgehend vom entsprechenden Nitril mittels Mikroorganismen der Gattungen *Rhodococcus, Amycolatopsis* und *Actinomadura*. Nachteilig bei diesem Verfahren ist, dass die eingesetzten Mikroorganismen der Gattung *Amycolatopsis* bei höheren Temperaturen inaktiviert werden. Des weiteren besitzen diese Mikroorganismen eine geringe Toleranz gegenüber 3-Cyanpyridin und Nikotinsäureamid. Die beschriebenen Mikroorganismen der Gattung *Rhodococcus* haben einen hohen K_{M}-Wert bezüglich 3-Cyanpyridin und eine geringe Temperaturstabilität. Demnach ist dieses Verfahren für ein grosstechnisches Verfahren nicht wirtschaftlich.

Aufgabe der vorliegenden Erfindung war es, Mikroorganismen bereit zu stellen, die stabiler sind, einen tieferen Kₘ-Wert für beispielsweise das Substrat 3-Cyanpyridin aufweisen und demnach für ein wirtschaftlicheres Verfahren zur Herstellung von Amiden eingesetzt werden können, bei dem das entsprechende Amid in sehr guter Ausbeute und in guter Reinheit isoliert werden kann.

Diese Aufgabe wird mit den Mikroorganismen nach Anspruch 1, mit dem Enzym nach Anspruch 6 oder 7 und mit dem Verfahren nach Anspruch 8 gelöst.

Die erfindungsgemässen Mikroorganismen sind durch geeignete Selektion beispielsweise aus Bodenproben, Schlamm oder Abwasser unter Zuhilfenahme üblicher mikrobiologischer Techniken erhältlich. Zweckmässig werden die Mikroorganismen durch Anzucht mit einem Pyridinaldoxim der allgemeinen Formel 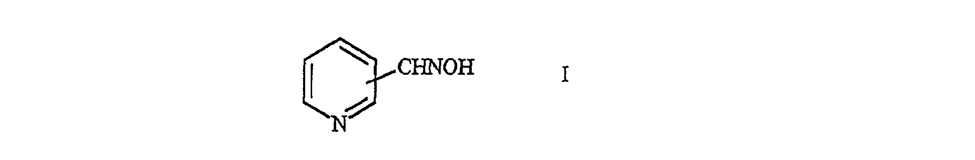 oder mit Nitrilen als C-Quelle in Gegenwart von Cobaltionen und beispielsweise Hefeextrakt und/oder Ammoniumsalzen selektiert. Aus den erhaltenen Kulturen werden dann jene Mikroorganismen selektiert, die befähigt sind, eine Konzentration an Acetonitril von wenigstens 3 M zu tolerieren und die in der Lage sind, Nitrile, wie beispielsweise 3-Cyanpyridin und Acetonitril, in das entsprechende Amid zu überführen.

Als Pyridinaldoxime können Pyridin-2-, Pyridin-3- oder Pyridin-4-aldoxim verwendet werden.

Zur Selektion geeignete Nitrile sind insbesondere auch die, die bei der späteren Biotransformation als Substrat eingesetzt werden sollen, z. B. Acetonitril (Essigsäurenitril), Propionitril, Butyronitril, Crotonsäurenitril, Adipinsäurenitril und Malonsäurenitril.

Als Quelle für die Cobaltionen werden sog. "Cobaltionen generierende Cobaltverbindungen" bevorzugt, beispielsweise Go²⁺- oder Co³⁺-Salze wie Cobaltchloride, Cobaltsulfate und Cobaltacetate. Vorzugsweise wird als Cobaltverbindung ein Co²⁺-Salz wie z.B. CoCl₂ eingesetzt. Die Anzucht kann jedoch auch zusammen mit metallischem Cobalt oder mit anderen Cobaltverbindungen erfolgen. Üblicherweise werden Cobalt oder Cobaltverbindungen in einer Menge von 1 bis 30 mg/L, vorzugsweise von 1 bis 20 mg/L im Anzuchtmedium eingesetzt.

Als Ammoniumsalze können beispielsweise Ammoniumphosphate wie (NH₄)₂HPO₄ oder (NH₄)H₂PO₄ eingesetzt werden.

Die Mikroorganismen werden vor der eigentlichen Biotransformation in geeigneten Medien kultiviert. Geeignete Kulturmedien sind beispielsweise die in den Tabellen 3 oder 5 beschriebenen Medien.

Üblicherweise erfolgt die Anzucht bei einer Temperatur von 20 bis 40 °C und bei einem pH-Wert zwischen 5 und 8, vorzugsweise bei einer Temperatur von 25 bis 35 °C und bei einem pH-Wert zwischen 6 und 7,5.

Während der Anzucht werden zweckmässig die wirksamen Enzyme, die Nitrilhydratasen, durch Zusatz eines Enzyminduktors induziert.

Als Enzyminduktoren können gesättigte oder ungesättigte aliphatische Nitrile oder die entsprechenden Amide eingesetzt werden. Als aliphatische Nitrile können alle C₂₋₇-Alkannitrile, wie beispielsweise Butyronitril, Isobutyronitril, Valeriansäurenitril oder Isovaleriansäurenitril, oder C₃₋₇-Alkennitrile, wie beispielsweise Methacrylnitril oder Crotonsäurenitril, eingesetzt werden. Als aliphatische Amide können alle C₂₋₇-Alkanamide wie beipielsweise Butyramid, Isobutyramid, Valeriansäureamid oder Propionsäureamid, oder C₃₋₇-Alkenamide, wie beispielsweise Methacrylamid oder Crotonsäureamid, verwendet werden. Die bevorzugten Enzyminduktoren sind Methacrylamid, Butyramid, Isobutyramid, Valeriansäureamid, Methacrylnitril, Crotonsäureamid, Butyronitril und Isobutyronitril. Besonders bevorzugt wird als Enzyminduktor Methacrylnitril eingesetzt.

Die erfindungsgemässen Mikroorganismen tolerieren eine Konzentration an Acetonitril von mindestens 3 M. Darunter ist zu verstehen, dass die Enzymaktivität nach 1 stündiger Inkubation mit 3 M Acetonitril in 0,1 M Kaliumphosphatpuffer bei pH 7,0 und 20 °C stabil ist, d.h. dass der Aktivitätsverlust maximal 10% beträgt. Bevorzugte Mikroorganismen tolerieren eine Acetonitril-Konzentration von mindestens 6 M für 1 Stunde unter den oben genannten Bedingungen bei einem Aktivitätsverlust von maximal 50%. Besonders bevorzugte Mikroorganismen tolerieren eine Acetonitril-Konzentration von mindestens 9 M für 1 Stunde unter den genannten Bedingungen bei einem Aktivitätsverlust von maximal 70%.

Bei ganz besonders bevorzugten Mikroorganismen ist die Enzymaktivität selbst nach einer mehrminütigen Inkubation mit 15 M und 19 M Acetonitril (entsprechend reinem Acetonitril) stabil. So beträgt der Verlust der Enzymaktivität bei Inkubation mit 15 M Acetonitril nach 10 min weniger als 10%.

Die erfindungsgemäßen Mikroorganismen weisen eine hohe thermische Stabilität auf, d.h. eine höhere Stabilität bei hohen Temperaturen als die bisher bekannten Mikroorganismen. Bevorzugt beträgt der Verlust der Enzymaktivität der erfindungsgemäßen Mikroorganismen nach 1 stündiger Inkubation in 0,1 M Kaliumphosphatpuffer, pH 7,0, bei 60 °C maximal 10% und der Verlust der Enzymaktivität nach 2 stündiger Inkubation unter den genannten Bedingungen beträgt maximal 40%.

Unter Enzymaktivität wird hier die Nitrilhydratase-Aktivität, insbesondere die Nitrilhydratase-Aktivität bezüglich 3-Cyanpyridin als Substrat, verstanden.

Weitere Eigenschaften der erfindungsgemässen Mikroorganismen sind eine hohe Toleranz bezüglich des bevorzugt eingesetzten Substrates 3-Cyanpyridin und bezüglich des daraus gebildeten Produktes Nikotinsäureamid und ein niedriger K_{M}-Wert bezüglich 3-Cyanpyridin. Eine besonders hervorragende Eigenschaft ist auch, dass sie Acetamid in einer höheren Konzentration akkumulieren können als der Konzentration einer bei 30 °C gesättigten Acetamid-Lösung (ca. 220-230 g Acetamid in 100 mL Wasser) entspricht. Erfindungsgemässe Mikroorganismen sind der Stamm *Rhodococcus sp.* FZ4 und dessen funktionell äquivalente

Varianten und Mutanten. Unter funktionell äquivalenten Varianten und Mutanten werden solche Varianten und Mutanten verstanden, die Acetonitril in Konzentrationen von mindestens 3 M tolerieren. Ganz besonders bevorzugt sind "Pigment-negative" *Rhodococcus-*Stämme, d.h.Stämme, die keine rote Farbe besitzen, die zur Verfärbung des gewünschten Produktes führen kann. Solche Stämme lassen sich gegebenenfalls leicht aus pigmentbildenden Mikroorganismen durch Mutagenese mittels UV-Bestrahlung oder mutagenen Chemikalien erzeugen.

Der Stamm *Rhodococcus sp*. FZ4 wurde am 11. Juli 2000 bei der Deutschen Sammlung von Mikroorganismen und Zellkulturen GmbH (DSMZ), Mascheroder Weg 1b, D-38124 Braunschweig gemäss Budapester Vertrag unter der Hinterlegungsnummer DSM 13597 hinterlegt. Dieser Mikroorganismus konnte aufgrund seiner Identifizierungsdaten keiner der bisher bekannten *Rhodococcus*-Spezies zugeordnet werden und wurde daher einer neuen Spezies zugeordnet.

Die funktionell äquivalenten Varianten und Mutanten des Stammes *Rhodococcus sp*. FZ4 können entweder durch spontane Mutation oder beispielsweise durch UV-Bestrahlung oder mutagene Chemikalien gebildet werden. Bevorzugte Varianten und Mutanten des Stammes *Rhodococcus sp*. FZ4 sind "Pigment-negativ", d. h. sie besitzen keine rote Farbe, die zur Verfärbung des gewünschten Produktes fuhren kann.

Das Enzymextrakt kann beispielsweise durch Aufschliessen der Mikroorganismen gewonnen werden, wie beispielsweise mittels Ultraschall, French-Press oder mittels der Lysozym-Methode.

Die erfindungsgemässen Enzyme mit Nitrilhydratase-Aktivität sind aus den oben beschriebenen Mikroorganismen erhältlich. Vorzugsweise sind sie erhältlich aus den Mikroorganismen der Gattung *Rhodococcus*, insbesondere aus dem Mikroorganismus *Rhodococcus sp*. FZ4 (DSM 13597).

Diese Enzyme weisen insbesondere folgende Eigenschaften auf
a) einen K_{M}-Wert für das Substrat Acetonitril von 2,84 ± 1,00 mM und für das Substrat 3-Cyanpyridin von 80,5 ± 15,0 mM, jeweils in 0,05 M Kaliumphosphatpuffer, pH 7,0, bei 20 °C;
b) ein pH-Optimum von pH 6,5 ± 1,0 bei 20 °C in 0,05 M Kaliumphosphatpuffer;
c) ein natives Molekulargewicht von 465 ± 50 kDa, bestimmt durch HPLC.

Die eigentliche Biotransformation kann mit den oben beschriebenen Mikroorganismen, mit einem Enzymextrakt dieser Mikroorganismen oder mit dem isolierten Enzym durchgeführt werden. Vorzugsweise wird die Biotransformation mit dem Mikroorganismen *Rhodococcus sp*. FZ4 durchgeführt.

Als Substrate für die Biotransformation können Nitrile der allgemeinen Formel

R¹-CN II

eingesetzt werden.

In der allgemeinen Formel II bedeutet der Substituent R¹ eine C₁₋₆-Mkylgruppe, eine C₂₋₆-Alkenylgruppe oder eine Gruppe der allgemeinen Formel IV

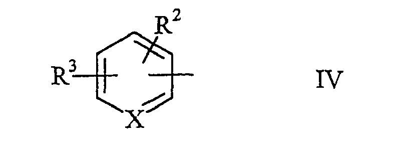

In der allgemeinen Formel IV bedeutet X ein Stickstoffatom oder -CH= und die Substituenten R² und R³ bedeuten unabhängig voneinander ein Wasserstoffatom, ein Halogenatom, eine C₁₋₆-Alkylgruppe oder eine C₂-₆-Alkenylgruppe.

Als C₁₋₆-Alkylgruppe kann Methyl, Ethyl, Propyl, Isopropyl, Butyl, tert-Butyl, Isobutyl, Pentyl und seine Isomere sowie Hexyl und seine Isomere verwendet werden. Als C₂-₆-Alkenylgruppe kann beispielsweise Vinyl, Allyl, 1-Propen-1-yl oder 1-Propen-2-yl eingesetzt werden.

Als Halogenatom kann F, Cl, Br oder I verwendet werden.

Bevorzugte Vertreter der Nitrile der allgemeinen Formel II sind Acetonitril, Butyronitril, Acrylnitril, Propionitril, Crotonsäurenitril, 2-Cyanpyridin, 3-Cyanpyridin, 4-Cyanpyridin, Benzonitril, Fluorbenzonitril, Chlorbenzonitril und Brombenzonitril. Die am meisten bevorzugten Substrate sind Acetonitril und 3-Cyanpyridin.

Bevorzugt wird die Biotransformation unter einmaliger oder kontinuierlicher Substratzugabe durchgeführt. Wie dem Fachmann bekannt, ist die einzusetzende Substratkonzentration abhängig von der Löslichkeit des einzusetzenden Substrates.

Bevorzugt wird das Verfahren mit ruhenden (nicht wachsenden) Zellen durchgeführt.

Als Medien für die Biotransformation können die in der Fachwelt üblichen eingesetzt werden, beispielsweise niedermolare Phosphatpuffer, HEPES-Puffer, Citrat-Puffer und Borat-Puffer. Unter niedermolaren Phosphatpuffern wird bevorzugt ein 0,01 bis 0,5 M Phosphatpuffer, besonders bevorzugt ein 0,05 bis 0,25 M Phosphatpuffer verstanden.

Bevorzugt wird die Biotransformation bei einer Temperatur von 5 bis 50 °C, besonders bevorzugt bei einer Temperatur von 20 bis 40 °C, durchgeführt. Der bevorzugte pH-Wert liegt zwischen 5 und 10, der besonders bevorzugte zwischen 6 und 7,5.

Nach der Umsetzung des Nitrils der allgemeinen Formel II können dann die entsprechenden Amide der allgemeinen Formeln

R¹-CONH₂ III

worin R¹ die bereits genannte Bedeutung hat, gegebenenfalls nach Abtrennung der Zellen, durch übliche Aufarbeitungsmethoden wie beispielsweise Kristallisation oder Sprühtrocknung isoliert werden.

Ein weiterer Gegenstand der vorliegenden Erfindung ist die Verwendung der zuvor beschriebenen Mikroorganismen, insbesondere der Gattung *Rhodococcus*, zur Beseitigung von Acetonitril-Abfällen.

Acetonitril ist ein Lösungsmittel, das beispielsweise bei der HPLC eingesetzt wird, und schliesslich als Abfall entsorgt werden muss. Bei der erfindungsgemässen Beseitigung von Acetonitril-Abfällen kann das Acetonitril in einer Konzentration bis maximal 19 M, welches reinem Acetonitril entspricht, vorliegen. Vorteilhaft wird eine 0,25 bis 15,0 M, vorzugsweise eine 1 bis 10 M Acetonitril-Lösung bzw. -Suspension eingesetzt.

Vorteilhaft werden die Mikroorganismen zur Beseitigung von Acetonitril-Abfällen bei einer Temperatur von 5 bis 50 °C, vorzugsweise bei einer Temperatur von 20 bis 40 °C, eingesetzt. Der pH-Wert liegt vorteilhaft zwischen 5 und 10, vorzugsweise zwischen 6 und 8.

Die Umsetzungsdauer von Acetonitril zu Acetamid für die Abfallbeseitigung ist von der Acetonitril-Konzentration abhängig. Beispielsweise beträgt diese bei der Herstellung von 9,5 M Acetamid Lösung/Suspension ca. 2 Stunden, bei einem pH von 7,0 und einer Temperatur von ca. 20 °C.

Identifizierung des Stammes FZ4 (DSM 13597):
A) chemotaxonomische "markers":
   1. Diagnostische Aminosäure des Peptidoglycans: meso-Diaminopimelinsäure
   2. Mycolsäuren: Mycolsäuren mit einer Kettenlänge von C₄₀ bis C₄₈ sind vorhanden
   3. Fettsäuremuster: Es sind linear, gesättigte und ungesättigte Fettsäuren vorhanden sowie ein hoher Anteil an Tuberculostearinsäure. Anhand der Fettsäuremuster wurde der Stamm FZ4 als zur Gattung *Rhodococcus* angehörend identifiziert.
B) konventionelle "markers":
   Das makroskopische Erscheinungsbild und die Morphologie der Zellen des Stammes FZ4 waren zu *Rhodococcus rhodochrous* ähnlich. Die Kolonien des Stammes FZ4 sind lachsrot (RAL 3022) und junge Kulturen entwickelten verzweigte Hyphen, die sich zu Stäbchen und Kokken entwickelten.

   Auf Grund der chemotaxonomischen und konventionellen "markers" wurde der Stamm FZ4 als zur Spezies *Rhodococcus rhodochrous* angehörend identifiziert, jedoch mit einem niedrigen Korrelationsfaktor.
C) Analyse der ersten 500 Basen der 16s rDNA
   Die Sequenz der ersten 500 Basen der 16S rDNA zeigt eine Ähnlichkeit von nur 97,7% zu der des typischen Vertreter-Stamms für die Spezies *Rhodococcus rhodochrous, Rhodococcus rhodochrous* DSM 43241, und von 99,1% zu einem anderen *Rhodococcus rhodochrous* Referenz-Stamm. Da die Ähnlichkeit der Sequenz der ersten 500 Basen der 16S rDNA des Stammes FZ4 zu der des Stammes *Rhodococcus rhodochrous* DSM 43241 unter 99,5% lag, kann der Stamm FZ4 nicht als zu der Spezies *Rhodococcus rhodochrous* gehörend identifiziert werden.

Der Stamm FZ4 wurde daher als neue Spezies innerhalb der Gattung *Rhodococcus* identifiziert.

### Abbildungen:

Figur 1: Dargestellt ist die Biotransformation von Acetonitril zu Acetamid mit ruhenden Zellen von *Rhodococcus sp*. FZ4.
Figur 2: Dargestellt ist das Temperaturoptimum der Nitrilhydratase-Aktivität von *Rhodococcus sp.* FZ4 in ruhenden Zellen.
Figur 3: Dargestellt ist die thermische Stabilität der Nitrilhydratase-Aktivität von *Rhodococcus sp*. FZ4 in ruhenden Zellen.
Figur 4: Dargestellt ist das pH-Optimum der Nitrilhydratase-Aktivität von *Rhodococcus sp*. FZ4 in ruhenden Zellen.
Figur 5: Dargestellt ist dies pH-Stabilität der Nitrilhydratase-Aktivität von *Rhodococcus sp*. FZ4 in ruhenden Zellen.
Figur 6: Dargestellt ist 3-Cyanpyridin-Toleranz der Nitrilhydratase-Aktivität von *Rhodococcus sp.* FZ4 in ruhenden Zellen.
Figur 7: Dargestellt ist Nikotinsäureamid-Toleranz der Nitrilhydratase-Aktivität von *Rhodococcus sp*. FZ4 in ruhenden Zellen.
Figur 8: Dargestellt ist der Einfluss der Acetonitril-Konzentration auf die Nitrilhydratase-Aktivität von *Rhodococcus sp*. FZ4 in ruhenden Zellen bei der Biotransformation von Acetonitril zu Acetamid.
Figur 9: Dargestellt ist der Einfluss der Acetonitrilkonzentration auf die Nitrilhydratase-Aktivität von *Rhodococcus sp*. FZ4 in ruhenden Zellen.
Figur 10: Dargestellt ist die Nitrilhydrataseaktivität von *Rhodococcus sp*. FZ4 in ruhenden Zellen in Abhängigkeit von der 3-Cyanpyridin-Konzentration.
Figur 11: Dargestellt ist die logarithmsche Auftragung der Molekulargewichte der Nitrilhydratase und der Referenzproteine gegen die jeweilige HPLC Retentionszeit.
Figur 12: Dargestellt ist die logarithmsche Auftragung der Molekulargewichte der Nitrilhydratase-Untereinheiten und der Referenzproteine gegen den jeweiligen SDS Page RF-Wert.
Figur 13: Dargestellt ist die Aktivität der gereinigten Nitrilhydratase aus *Rhodococcus sp*. FZ4 in Abhängigkeit von der 3-Cyanpyridin-Konzentration.
Figur 14: Dargestellt ist die Aktivität der gereinigten Nitrilhydratase aus *Rhodococcus sp*. FZ4 in Abhängigkeit von der Acetonitril-Konzentration.
Figur 15: Dargestellt ist die thermische Stabilität der gereinigten Nitrilhydratase aus *Rhodococcus sp*. FZ4.
Figur 16: Dargestellt ist das pH-Optimum der gereinigten Nitrilhydratase aus *Rhodococcus sp*. FZ4.
Figur 17: Dargestellt ist die pH-Stabilität der gereinigten Nitrilhydratase aus *Rhodococcus sp*. FZ4.

### Beispiel 1

### Bestimmung der Nitrilhydratase-Aktivität

Zur Bestimmung der Nitrilhydratase-Aktivität wurde eine Reaktionsmischung bestehend aus 3-Cyanpyridin (1,0 M; 1,0 mL), Kaliumphosphatpuffer (0,1 M, pH 7,0; 0,5 mL) und Zellsuspension (0,5 mL) unter Rühren bei 20 °C für 5 min inkubiert. Die Reaktion wurde durch Zugabe von HC1 (5 M; 0,1 mL) gestoppt. Nach Filtrieren (0,2 µm Filter) der Reaktionsmischung wurde die Menge des gebildeten Nikotinsäureamids mittels HPLC (Waters Spherisorb 5 µ ODS2 (4,6 × 150 mm); KH₂PO₄/H₃PO₄ (10 mM; pH 2,8)/Acetonitril = 9:1 (v/v); 1 mL/min; 230 nm) bestimmt. Die Gesamtaktivität wird ausgedrückt als µmol gebildetes Nikotinsäureamid/(min × mL) und die spezifische Aktivität wird ausgedrückt als µmol gebildetes Nikotinsäureamid/(min × mL × OD₆₁₀ₙₘ).

### Beispiel 2

### Isolation des Stamms Rhodococcus sp. FZ4 (DSM 13597)

In Arch. Microbiol. 1998, 170, 85-90 ist beschrieben, dass der Stamm *Rhodococcus sp*. YH3-3 (TPU 3453) 3-Pyridinaldoxim über 3-Cyanpyridin und Nikotinsäureamid zu Nikotinsäure metabolisiert. In diesem Fall wurde die Aldoximdehydratase-Aktivität sowie die Nitrilhydratase- und Amidase-Aktivität des Stammes *Rhodococcus sp.* YH3-3 (TPU 3453) sowohl von verschiedenen Aldoximen wie auch Nitrilen induziert.

Verschiedene Bodenproben wurden in das Anreicherungsmedium gemäss Tabelle 1 inokuliert und 7 bis 10 Tage bei 37 °C inkubiert. Die so erhaltenen Kulturen wurden in das gleiche Medium überimpft und nochmals 7 bis 10 Tage bei 37 °C kultiviert. Diese Prozedur wurde dreimal wiederholt. Anschliessend wurden die Kulturen verdünnt und ausplattiert. Nach 5 tägiger Inkubation der Platten bei 37 °C, wurden einzelne Kolonien erhalten. Die einzelnen Kolonien wurden auf das Vorhandensein einer Nitrilhydratase-Aktivität gemäss Beispiel 1 getestet. Auf diese Weise wurde der Stamm *Rhodococcus sp*. FZ4 (DSM 13597) isoliert. Anstelle von 3-Pyridinaldoxim können auch die Nitrile Acetonitril, Propionitril, Butyronitril, Crotonsäurenitril, Adipinsäurenitril und Malonsäurenitril als C-Quelle eingesetzt werden.

**Tabelle 1: Anreicherungsmedium**

| **Komponenten** | **Konzentration [g/L]** |
|---|---|
| 3-Pyridinaldoxim | 3,0 oder 1,0 |
| (NH₄)₂HPO₄ | 2,0 |
| KH₂PO₄ | 2,0 |
| NaC1 | 1,0 |
| MgSO₄ × 7 H₂O | 0,2 |
| CoCl₂ × 6 H₂O | 0,01 |
| Hefeextrakt | 0,2 |

| | |
|---|---|
| mit Wasser auf 1 L aufgefüllt (pH 7,0) | |

### Beispiel 3

### Einfluss von Cofaktoren während der Anzucht auf die Nitrilhydratase-Aktivität von Rhodococcus sp. FZ4

Der Stamm *Rhodococcus sp*. FZ4 (DSM 13597) wurde in das Vorkulturmedium gemäss Tabelle 2 inokuliert und 1 bis 2 Tage bei 28°C unter Schütteln inkubiert. Die Vorkultur wurde in das Basalmedium gemäss Tabelle 3, welches entweder CoCl₂ oder FeSO₄ enthielt, überimpft und 2 bis 3 Tage bei 28 °C unter Schütteln kultiviert. Die Nitrilhydratase-Aktivität wurde gemäss Beispiel 1 bestimmt. Die Ergebnisse sind in Tabelle 4 zusammengestellt. Eine Nitrilhydratase-Aktivität war nur vorhanden, wenn *Rhodococcus sp.* FZ4 in Gegenwart von Cobalt kultiviert wurde.

**Tabelle 2: Vorkulturmedium**

| **Komponenten** | **Konzentration [g/L]** |
|---|---|
| Pepton | 5,0 |
| Fleischextrakt | 5,0 |
| NaCl | 2,0 |
| Hefeextrakt | 0,5 |

| | |
|---|---|
| mit Wasser auf 1 L aufgefüllt (pH 7,0) | |

**Tabelle 3: Basalmedium**

| **Komponenten** | **Konzentration [g/L]** |
|---|---|
| Hefeextrakt | 2,0 |
| Pepton | 0,2 |
| L-Glutamat, Natriumsalz | 15,0 |
| MgSO₄ × 7 H₂O | 0,5 |
| CoCl₂ × 6 H₂O (oder FeSO₄ × 7 H₂O) | 0,004 |
| Crotonamid | 5,0 |

| | |
|---|---|
| mit Wasser auf 1 L aufgefüllt (pH 6,8) | |

**Tabelle 4:**

| **Einfluss von Cofaktoren während der Anzucht auf die Nitrilhydratase-Aktivität** | | | |
|---|---|---|---|
| **Cofaktor** | **Wachstum [OD_{610 nm}]** | **Gesamtaktivität [µmol/(min × mL)** | **Spezifische Aktivität [µmol/(min × mL × OD₆₁₀ₙₘ)]** |
| FeSO₄ | 2,86 | 0,989 | 0,346 |
| CoCl₂ | 2,79 | 38,1 | 13,1 |

### Beispiel 4

### Einfluss von Induktoren während der Anzucht auf die Nitrilhydratase-Aktivität von Rhodococcus sp. FZ4

Der Stamm *Rhodococcus sp*. FZ4 (DSM 13597) wurde in das Vorkulturmedium gemäss Tabelle 2 inokuliert und 1 bis 2 Tage bei 28 °C unter Schütteln inkubiert. Die Vorkultur wurde in das Kulturmedium gemäss Tabelle 5, welches unterschiedliche Induktoren enthielt, überimpft und 3 Tage bei 28 °C unter Schütteln kultiviert. Die Nitrilhydratase-Aktivität wurde gemäss Beispiel 1 bestimmt. Die Ergebnisse sind in Tabelle 6 zusammengestellt. Die Nitrilhydratase aus *Rhodococcus sp.* FZ4 wurde während der Anzucht nur in Gegenwart eines Induktors exprimiert.

**Tabelle 5: Kulturmedium**

| **Komponenten** | **Konzentration [g/L]** |
|---|---|
| Hefeextrakt | 1,0 |
| Natriumcitrat | 10,0 |
| Malzextrakt | 15,0 |
| Induktor | 0,2% (w/v) |
| KH₂PO₄ | 2,0 |
| MgSO₄ × 7 H₂O | 0,5 |
| CoCl₂ × 6 H₂O | 0,015 |

| | |
|---|---|
| mit Wasser auf 1 L aufgefüllt (pH 7,0). | |

**Tabelle 6:**

| **Einfluss von Induktoren auf die Nitrilhydratase-Aktivität** | | | |
|---|---|---|---|
| **Induktor** | **Wachstum [OD₆₁₀ₙₘ]** | **Gesamtaktivität [µmol/(min × mL)** | **Spezifische Aktivität [µmol/(min × mL × OD₆₁₀ₙₘ)]** |
| Methacrylamid | 4,55 | 569 | 125 |
| Isobutyramid | 3,55 | 387 | 109 |
| Butyramid | 4,7 | 344 | 73,2 |
| Methacrylnitril | 4,61 | 330 | 71,5 |
| Crotonamid | 9,32 | 558 | 59,9 |
| Butyronitril | 5,26 | 307 | 58,4 |
| Valeriansäureamid | 5,61 | 322 | 57,5 |
| Isobutyronitril | 5,24 | 273 | 52,1 |
| Crotonsäurenitril | 8,24 | 407 | 49,4 |
| Propionsäureamid | 5,17 | 149 | 28,7 |
| Valeronitril | 3,70 | 120 | 32,4 |
| Isocapronsäurenitril | 4,72 | 134 | 28,5 |
| Isovaleronitril | 3,22 | 74,7 | 23,2 |
| Capronsäurenitril | 4,54 | 104 | 23,0 |
| Propionitril | 4,72 | 95,8 | 20,3 |
| Acrylamid | 5,17 | 60,5 | 11,7 |
| 3-Pentennitril | 4,62 | 62 | 13,4 |
| ε-Caprolactam | 4,44 | 40,3 | 9,08 |
| Benzonitril | 5,62 | 40,3 | 7,17 |
| Pikolinsäureamid | 3,80 | 26,1 | 6,87 |
| - | 4,2 | 27,7 | 6,59 |
| Cyanacetamid | 4,75 | 30,9 | 6,50 |
| Acetamid | 4,37 | 21,6 | 4,98 |
| Acetonitril | 4,46 | 18,4 | 4,13 |
| 3-Cyanpyridin | 5,54 | 12,3 | 2,21 |
| IsoNikotinsäureamid | 5,03 | 10,9 | 2,17 |
| Benzamid | 3,88 | 8,24 | 2,12 |
| Acrylnitril | 3,27 | 5,91 | 1,81 |
| Nikotinsäureamid | 3,55 | 4,94 | 1,39 |
| Harnstoff | 6,16 | 5,66 | 0,918 |

### Beispiel 5

### Anzucht von Rhodococcus sp. FZ4

Der Stamm *Rhodococcus sp.* FZ4 wurde in das Vorkulturmedium gemäss Tabelle 2 inokuliert und 1 bis 2 Tagen bei 28 °C unter Schütteln inkubiert. Die Vorkultur wurde in das Kulturmedium gemäss Tabelle 5 mit 6 g/L Methacrylamid als Induktor überimpft und 3 Tage bei 28°C unter Schütteln kultiviert. Nach 48 h wurde zusätzliches Methacrylamid (0,2% (v/v)) zugefüttert.

In den Beipielen 6 bis 13 wurden ruhende Zellen von *Rhodococcus sp*. FZ4 eingesetzt.

### Beispiel 6

### Substratspezifität der Nitrilhydratase-Aktivität von Rhodococcus sp. FZ4

Die Nitrilhydratase-Aktivität von *Rhodococcus sp*. FZ4 bezüglich unterschiedlicher Substrate wurde gemäss Beispiel 1 bestimmt, nur dass statt 3-Cyanpyridin das entsprechende Substrat eingesetzt wurde und die HPLC Bedingungen dem eingesetzten Substrat entsprechend abgeändert wurden. Die Substratspezifität der Nitrilhydratase-Aktivität von *Rhodococcus sp.* FZ4 im Vergleich zu der Substratspezifität der Nitrilhydratase-Aktivität von *Rhodococcus rhodochrous* J1 ist in Tabelle 7 zusammengestellt.

**Tabelle 7: Vergleich der Substratspezifitäten der Nitrilhydratase-Aktivität von Rhodococcus sp. FZ4 und von Rhodococcus rhodochrous J1**

| | *Rhodococcus sp.* FZ4 | *Rhodococcus rhodochrous* J1 |
|---|---|---|
| **Substrat** | **Relative Aktivität [%]** | **Relative Aktivität [%]** |
| Acetonitril | 646 | 0 (Nitrilhydratase A) |
| | | 115 (Nitrilhydratase B) |
| Acrylnitril | 498 | 478 |
| Butyronitril | 466 | 26 |
| Propionitril | 412 | 435 |
| 3-Cyanpyridin | 100 | 100 |
| 4-Cyanpyridin | 98,4 | 70 |
| Crotonsäurenitril | 92,1 | 78 |
| Benzonitril | 41,7 | 27 |
| 2-Cyanpyridin | 39,3 | 45 |
| m-Chlorbenzonitril | 39,3 | 43 |
| p-Chlorbenzonitril | 8,25 | 13 |
| Methacrylnitril | 3,64 | 87 |
| o-Chlorbenzonitril | 0 | 2,8 |

### Beispiel 7

### Temperaturoptimum und thermische Stabilität der Nitrilhydratase-Aktivität von Rhodococcus sp. FZ4

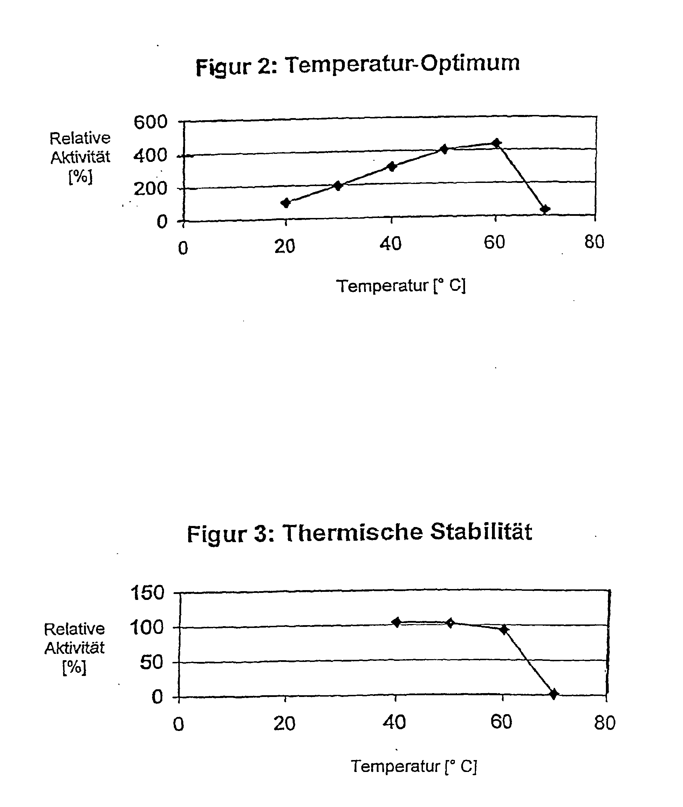

Die Nitrilhydratase-Aktivität wurde gemäss Beispiel 1 bei unterschiedlichen Temperaturen im Bereich von 20 bis 70 °C bestimmt. Das Temperaturoptimum für die Nitrilhydratase-Aktivität lag bei 60 °C (Fig. 2).
Um die thermische Stabilität der Nitrilhydratase-Aktivität zu bestimmen, wurde die Zellsuspension 15 min bei unterschiedlichen Temperaturen im Bereich von 40 bis 70 °C inkubiert. Danach wurde die Nitrilhydratase-Aktivität gemäss Beispiel 1 bei 20 °C bestimmt.
Die Nitrilhydratase-Aktivität nach 15 minütiger Inkubation bei Temperaturen im Bereich von 40 bis 60 °C entsprach der ursprünglichen Nitrilhydratase-Aktivität (Fig. 3).

### Beispiel 8

### pH-Optimum und pH-Stabilität der Nitrilhydratase-Aktivität von Rhodococcus sp. FZ4

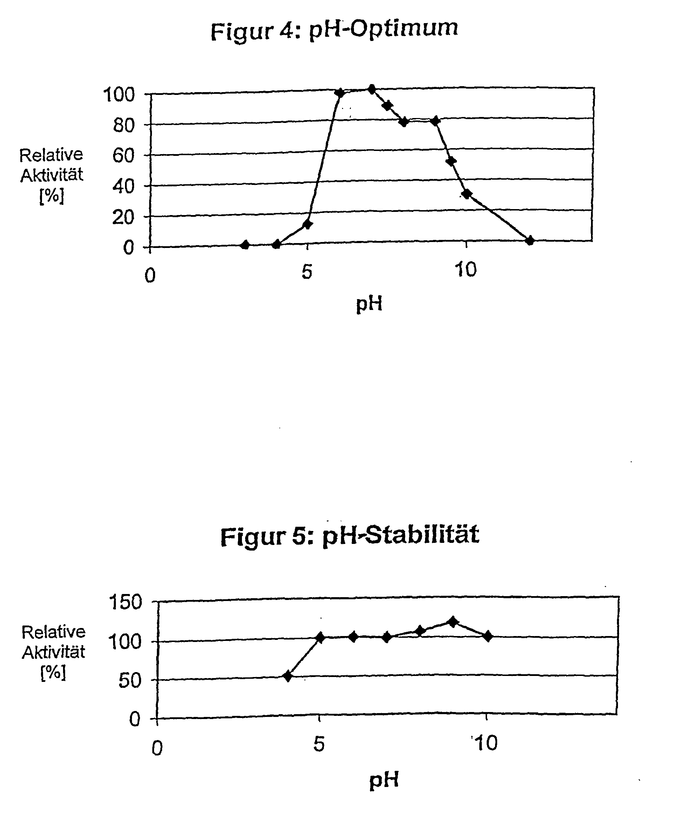

Die Nitrilhydratase-Aktivität wurde gemäss Beispiel 1 bei unterschiedlichen pH-Werten im Bereich von 3 bis 12 unter Verwendung unterschiedlicher Puffer (0,1 M) bestimmt. Das pH-Optimum der Nitrilhydratase-Aktivität lag zwischen 6 und 7 (Fig. 4).
Um die pH-Stabilität der Nitrilhydratase-Aktivität zu bestimmen, wurde die Zellsuspension bei unterschiedlichen pH-Werten im Bereich von 4 bis 10 für 24 h bei 20 °C inkubiert. Danach wurde die Zellsuspension zentrifugiert. Die abgetrennten Zellen wurden gewaschen und in Kaliumphosphatpuffer (0,1 M; pH 7,0) resuspendiert. Die Nitrilhydratase-Aktivität wurde gemäss Beispiel 1 bestimmt. Die Nitrilhydratase-Aktivität nach 24 stündiger Inkubation bei pH-Werten im Bereich von 5 bis 10 entsprach annähernd der ursprünglichen Nitrilhydratase-Aktivität (Fig. 5).

### Beispiel 9

### Einfluss der 3-Cyanpyridin-Konzentration auf die Nitrilhydratase-Aktivität von Rhodococcus sp. FZ4

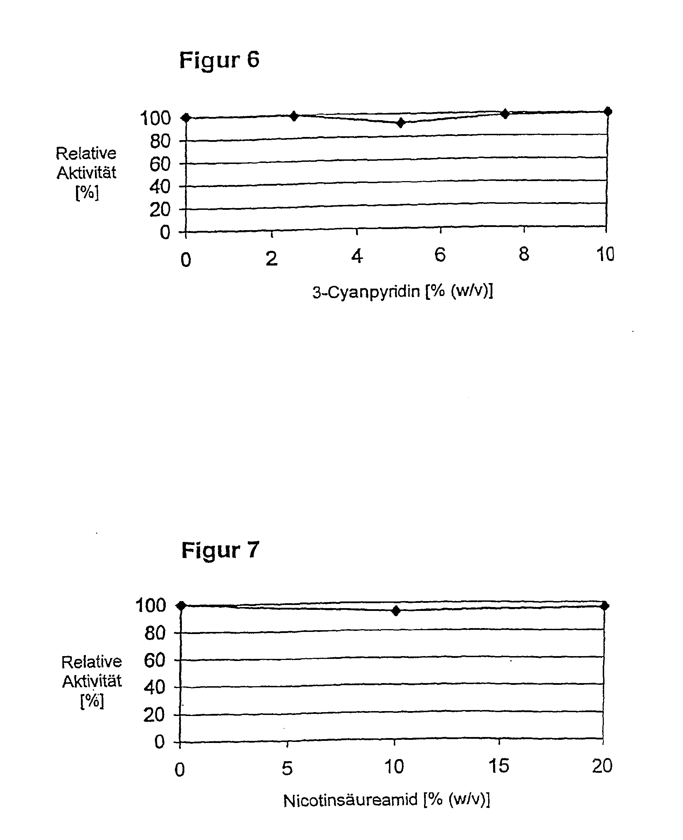

Eine Zellsuspension wurde für 60 min bei 20 °C bei unterschiedlichen 3-Cyanpyridin Konzentrationen im Bereich von 0 bis 10% (w/v) inkubiert. Die Zellen wurden abgetrennt, gewaschen und in Kaliumphosphatpuffer (0,1 M; pH 7,0) resuspendiert. Die Nitrilhydratase-Aktivität wurde gemäss Beispiel 1 bestimmt. Die Nitrilhydratase-Aktivität nach 60 minütiger Inkubation bei 3-Cyanpyridin-Konzentrationen im Bereich von 0 bis 20% (w/v) entsprach annähernd der ursprünglichen Nitrilhydratase-Aktivität (Fig. 6).

### Beispiel 10

### Einfluss der Nikotinsäureamid-Konzentration auf die Nitrilhydratase-Aktivität von Rhodococcus sp. FZ4

Eine Zellsuspension wurden für 24 h bei 20°C bei unterschiedlichen Nikotinsäureamid-Konzentration im Bereich von 0 bis 20% (w/v) inkubiert. Die Zellen wurden abgetrennt, gewaschen und in 0,1 M Kaliumphosphatpuffer (0,1 M; pH 7,0) resuspendiert. Die Nitrilhydratase-Aktivität wurde gemäss Beispiel 1 bestimmt. Die Nitrilhydratase-Aktivität nach 24 stündiger Inkubation bei Nikotinsäureamid-Konzentrationen im Bereich von 0 bis 20% (w/v) entsprach annähernd der ursprünglichen Nitrilhydratase-Aktivität (Fig. 7).

### Beispiel 11

### Bestimmung des K_{M}-Wertes für 3-Cyanpyridin bezüglich der Nitrilhydratase von Rhodococcus sp. FZ4

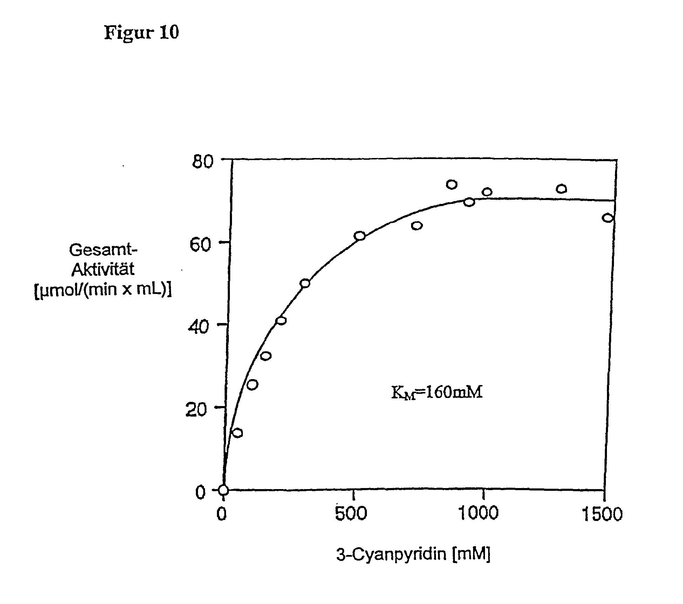

Eine Reaktionsmischung bestehend aus 3-Cyanpyridin (0,1-1,0 M; 1,0-1,8 mL), wässriger NaCl-Lösung (0,85% (w/v); 0,7 bis 0,1 mL), Kaliumphosphatpuffer (0,1 M; pH 7,0; 0,3 mL) und Zellsuspension (0,01 mL) wurde 10 min bei 30 °C unter Schütteln inkubiert. Das Gesamtvolumen der Reaktionsmischung lag je nach 3-Cyanpyridin-Konzentration zwischen 2,0 bis 2,2 mL. Die Reaktion wurde durch Zugabe von HC1 (2 M; 0,1 mL) gestoppt. Nach Zentrifugation (12 000 Upm; 5 min) der Reaktionsmischung wurde die Menge des gebildeten Nikotinsäureamids mittels HPLC gemäss Beispiel 1 bestimmt. Der K_{M}-Wert wurde zu 160 mM ermittelt (Fig. 10).

### Beispiel 12

### Vergleich der Nitrilhydratase-Aktivitäten von Rhododoccus sp. FZ4 mit den Nitrilhydratase-Aktivitäten der bekannten Mikroorganismen Amycolatopsis sp. NA40, Rhodococcus sp. GF270 und Rhodococcus rhodochrous J1

Die K_{M}-Werte für das Substrat 3-Cyanpyridin bezüglich der Nitrilhydratase-Aktivitäten von *Rhododococcus sp*. FZ4, *Rhodococcus sp*. GF270 (DSM 12211; WO 99/05306), *Amycolatopsis sp*. NA40 (DSM 11617; WO 99/05306) und *Rhodococcus rhodocrous* J1 wurden gemäss Beispiel 11 unter Verwendung des jeweiligen Mikroorganismus bestimmt.
Die K_{M}-Werte der Stämme *Amycolatopsis sp*.NA40 und *Rhodococcus sp*. FZ4 sind niedriger als die der anderen Mikroorganismen (Tabelle 8).

**Tabelle 8: Vergleich der K_{M}-Werte für das Substrat 3-Cyanpyridin**

| K_{M} [mM] | | | |
|---|---|---|---|
| *Rhodococcus sp*. FZ4 | *Rhodococcus sp*. GF270 | *Amycolatopsis sp*. NA40 | *Rhodococcus rhodochrous* J1 |
| 160 | > 200 | 41,7 | 200 |

Die thermische Stabilität der Nitrilhydratase-Aktivitäten von *Rhododococcus sp.* FZ4, *Rhodococcus sp*. GF270, *Amycolatopsis sp*. NA40 und *Rhodococcus rhodocrous* J1 wurde gemäss Beispiel 7 unter Verwendung des jeweiligen Mikroorganismus und der in Tabelle 9 angegebenen Inkubationsbedingungen bestimmt. Die Nitrilhydratase-Aktivität des Stammes *Rhodococcus sp*. FZ4 zeigte im Vergleich zu der Nitrilhydratase-Aktivität der anderen Mikroorganismen die grösste thermische Stabilität.

**Tabelle 9: Vergleich der thermischen Stabilität der Nitrilhydratase-Aktivitäten**

| | **Relative Aktivität [%]** | | | |
|---|---|---|---|---|
| **Inkubationsbedingungen** | *Rhodococcus sp*. FZ4 | *Rhodococcus sp*. GF270 | *Amycolatopsis sp*. NA40 | *Rhodococcus rhodochrous* J1 |
| 15 min bei | | | | |
| 50 °C | 100 | 100 | nb^{a} | 100 |
| 60 °C | 93 | 95 | nb^{a} | 80 |
| 70 °C | 2 | 5 | nb^{a} | 0 |
| 60 min bei | | | | |
| 20 °C | 100 | 100 | 100 | nb^{a} |
| 30 °C | 100 | 100 | 95 | nb^{a} |
| 40 °C | 100 | 100 | 80 | nb^{a} |
| 50 °C | 100 | 100 | 32 | nb^{a} |
| 60 °C | 100 | 89 | 0 | nb^{a} |
| 70 °C | 6 | 0 | 0 | nb^{a} |
| 60 °C für | | | | |
| 0 min | 100 | 100 | nb^{a} | nb^{a} |
| 30 min | 100 | 67-80 | nb^{a} | nb^{a} |
| 60 min | 92-100 | 52-68 | nb^{a} | nb^{a} |
| 120 min | 72-87 | 29-47 | nb³ | nb^{a} |

| | | | | |
|---|---|---|---|---|
| ^{a} nicht bestimmt. | | | | |

Der Einfluss der 3-Cyanpyridin-Konzentration auf die Nitrilhydratase-Aktivitäten von *Rhododococcus sp*. FZ4, *Rhodococcus sp*. GF270, *Amycolatopsis sp*. NA40 und *Rhodococcus rhodocrous* J1 wurde gemäss Beispiel 9 unter Verwendung des jeweiligen Mikroorganismus und der in Tabelle 10 angegebenen 3-Cyanpyridin Konzentrationen bestimmt. Die Nitrilhydratase-Aktivitäten der Stämme *Rhodococcus sp*. FZ4 und *Rhodococcus sp*. GF270 zeigen die höchste Toleranz gegenüber 3-Cyanpyridin.

**Tabelle 10: Vergleich des Einflusses der 3-Cyanpyridin Konzentration auf die Nitrilhydratase-Aktivität**

| | **Relative Aktivität [%]** | | | |
|---|---|---|---|---|
| **3-Cyanpyridin [% (w/v)]** | *Rhodococcus sp*. FZ4 | *Rhodococcus sp*. GF270 | *Amycolatopsis sp*. NA40 | *Rhodococcus rhodochrous* J1 |
| 0 | 100^{a} | 100^{a} | 100^{b} | 100^{b} |
| 2,5 | 100^{a} | 100^{a} | 74^{b} | nb^{c} |
| 5,0 | 100^{a} | 100^{a} | 56^{b} | 86^{b} |
| 7,5 | 100^{a} | 100^{a} | 47^{b} | nb^{c} |
| 10,0 | 100^{a} | 100^{a} | 16^{b} | 63^{b} |

| | | | | |
|---|---|---|---|---|
| ^{a} Inkubation für 60 min. ^{b} Inkubation für 15 min. ^{c} nicht bestimmt. | | | | |

Der Einfluss der Nikotinsäureamid Konzentration auf die Nitrilhydratase-Aktivitäten von *Rhododococcus sp.* FZ4, *Rhodococcus sp*. GF270, *Amycolatopsis sp*. NA40 und *Rhodococcus rhodocrous* J1 wurde gemäss Beispiel 9 unter Verwendung des jeweiligen Mikroorganismus und der in Tabelle 11 angegebenen Nikotinsäureamid-Konzentration bestimmt. Die Nitrilhydratase-Aktivitäten der Stämme *Rhodococcus sp*. FZ4 und *Rhodococcus sp*. GF270 zeigen die höchste Toleranz gegenüber Nikotinsäureamid (Tabelle 11).

**Tabelle 11: Vergleich des Einflusses der Nikotinsäureamid-Konzentration auf die Nitrilhydratase-Aktivitäten**

| | **Relative Aktivität [%]** | | | |
|---|---|---|---|---|
| **Nikotinsäureamid [% (w/v)]** | *Rhodococcus sp*. FZ4 | *Rhodococcus sp*. GF270 | *Amycolatopsis sp*. NA40 | *Rhodococcu*s *rhodochrous* J1 |
| 0 | 100 | 100 | 100 | nb^{a} |
| 10 | 100 | 100 | 55 | nb^{a} |
| 20 | 100 | 100 | 0 | nb^{a} |
| 30 | 100 | 100 | 0 | nb^{a} |

| | | | | |
|---|---|---|---|---|
| ^{a} nicht bestimmt. | | | | |

### Beispiel 13

### Biotransformation von 3-Cyanpyridin zu Nikotinsäureamid mit Rhodococcus sp. FZ4

Eine Lösung von 3-Cyanpyridin wurde in 42 Portionen (42 × 0,52 g = 21,8 g; 0,21 mol) zu einer Vorlage bestehend aus Zellsuspension (13,7 mg Zelltrockengewicht, 4 mL) und Kaliumphosphatpuffer (0,1 M; pH 6,0; 16 mL) zugefüttert. Die nächste Portion 3-Cyanpyridin wurde zum Reaktionsgemisch gegeben, wenn das 3-Cyanpyridin im Reaktionsgemisch quantitativ zu Nikotinsäureamid umgesetzt war. Das Reaktionsgemisch wurde im Verlauf der Reaktion fest. Insgesamt wurden 25,7 g (quantitative Ausbeute) Nikotinsäureamid gebildet.

### Beispiel 14

### Biotransformation von Acetonitril zu Acetamid mit Rhodococcus sp. FZ4

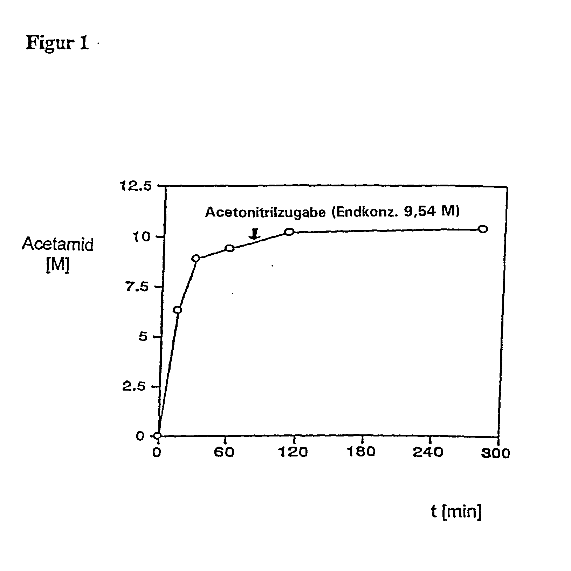

Acetonitril (5 mL; 95 mmol) wurde während 80 min zu einer Reaktionsmischung bestehend aus Kaliumphosphatpuffer (0,1 M; pH 7,0; 4,5 mL) und Zellsuspension (4,88 mg Zelltrockengewicht; 0,5 mL) bei 20 °C hinzugetropft. Die Nachreaktion erfolgte bei 20 °C unter Schütteln. Die Bildung von Acetamid während der Reaktion wurde mittels HPLC (Waters Spherisorp 5 µ ODS2 (4,6 × 150 mm); KH₂PO₄ (10 mM; pH 2,5)/Acetonitril = 99/1 (v/v); 1,0 mL/min; 210 nm) verfolgt. Innerhalb von 120 min wurde 6,14 g (quantitative Ausbeute) Acetamid gebildet, welches im Reaktionsmedium akkumuliert wurde (Fig. 1).

### Beispiel 15

### Einfluss der Acetonitrilkonzentration auf die Nitrilhydratase-Aktivität von Rhodococcus sp. FZ4 bei der Biotransformation von Acetonitril zu Acetamid

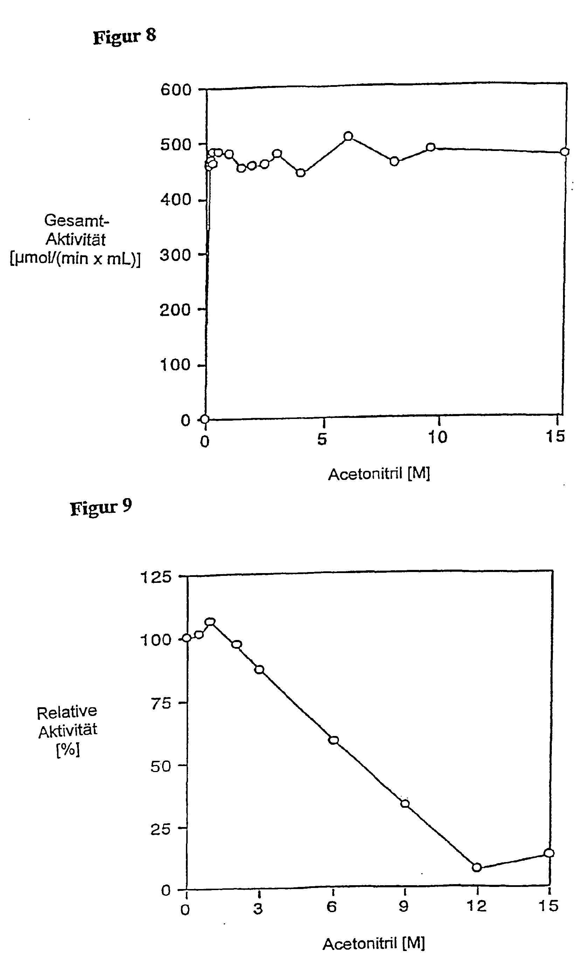

Ein Reaktionsgemisch bestehend aus Acetonitril (0,2-19,0 M; 1,0-1,6 mL), wässriger NaCl-Lösung (0,85% (w/v); 0,6-0,0 mL), Kaliumphosphatpuffer (0,1 M; pH 7,0; 0,3 mL) und Zellsuspension (0,1 mL) wurde 10 min bei 20 °C unter Schütteln inkubiert. Das Gesamtreaktionsvolumen betrug 2,0 mL. Die Reaktion wurde durch Zugabe von MeOH gestoppt. Das Reaktionsgemisch wurde zentrifugiert (12 000 Upm, 5 min) und die Menge gebildetes Acetamid wurde mittels HPLC gemäss Beipiel 14 bestimmt.
Die Nitrilhydratase-Aktivität war im Bereich von 0,1 bis 15 M Acetonitril annähernd konstant (Fig. 8).

### Beispiel 16

### Einfluss der Acetonitril Konzentration auf die Nitrilhydratase-Aktivität von Rhodococcus sp. FZ4.

Zellen wurden mit Acetonitril (0,0-15,0 M) in Kaliumphosphatpuffer (0,1 M, pH 7,0) bei 1 h bei 20 °C inkubiert. Die Zellsuspension wurde zentrifugiert (12 000 upm, 5 min) und die Zellen wurden in wässriger NaCl-Lösung (0.85% (w/v)) resuspendiert. Ein Reaktionsgemisch bestehend aus dieser Zellsuspension (0,1 mL), 3-Cyanpyridin (0,5 M; 1,0 mL), wässriger NaCl-Lösung (0,85% (w/v); 0,6 mL) und Kaliumphosphatpuffer (0,1 M; pH 7,0; 0,3 mL) wurde 5 min bei 30 °C unter Schütteln inkubiert. Die Reaktion wurde durch Zugabe von MeOH gestoppt. Das Reaktionsgemisch wurde zentrifugiert (12 000 Upm, 5 min) und die Menge gebildetes Acetamid wurde mittels HPLC gemäss Beipiel 14 bestimmt.
Die Nitrilhydratase-Aktivität war nach 1 stündiger Inkubation mit Acetonitril im Konzentrationsbereich von 0 bis 3 M annähernd konstant. Nach 1 stündiger Inkubation mit 6 M Acetonitril lag noch 60% der ursprünglichen Nitrilhydratase-Aktivität vor. Nach 1 stündiger Inkubation mit 9 M Acetonitril lag noch ca. 35% der ursprünglichen Nitrilhydratase-Aktivität vor (Fig. 9).

### Beispiel 17

### Erzeugung Pigment-negativer Mutanten von Rhodococcus sp. FZ4

Der Stamm *Rhodococcus sp*. FZ4 wurde in "Nutrient-Broth"-Medium (50 mL) inokubiert und bei 28 °C unter Schütteln inkubiert bis ein OD_{610 nm} von 6,29 erreicht war. Die Vorkultur (10 mL) wurde dann in "Nutrient Broth"-Medium (25 ml) überimpft und bei 28 °C unter Schütteln inkubiert bis ein OD₆₁₀ₙₘ von 1,90 erreicht war. Die so erhaltene Kultur (10 mL) wurde zentrifugiert (8 000 Upm, 5 min). Der Überstand wurde verworfen und der Zellniederschlag wurde in phosphatgepuffertem Kochsalz suspendiert. Die Zellsuspension wurde zentrifugiert (8 000 Upm, 5 min). Der Überstand wurde verworfen und der Zellniederschlag wurde in phosphatgepuffertem Kochsalz (5 mL) suspendiert. Die Zellsuspension wurde in eine Glaspetrischale (90 mm Durchmesser) überführt. Die Zellen wurden mit einer UV Lampe (15 W, 254 nm) aus 25 cm Entfernung für 17 min bestrahlt. Danach wurden die Zellen in doppelt konzentriertem "Nutrient Broth"-Medium bei 28 °C für 4 Tage unter Schütteln inkubiert. Die so erhaltene Kultur wurde um das 100fache verdünnt und 100 µl Aliquots hiervon wurden auf "plate count agar" ausplattiert und bei 28 °C inkubiert. Nahezu 150 Einzelkolonien wuchsen pro Platte. Die Platten wurden Tageslicht ausgesetzt, um die Ausbildung der roten Pigmente zu induzieren. Die Kolonien der Pigment-negativen Mutanten konnten leicht von denen der gefärbten Mutanten und der des roten Wildtypes unterschieden werden.

### Beispiel 18

### Reinigung der Nitrilhydratase aus Rhodococcus sp. FZ4

*Rhodococcus sp*. FZ4 wurde gemäss Beispiel 3 in einem 2 L Fermentor angezogen. Die Kultur wurde zentrifugiert und der Zellniederschlag wurde in wässriger NaCl-Lösung (0,85% (w/v)) resuspendiert. Die Zellsuspension wurde in Kaliumphosphatpuffer (0,1 M; pH 7,0) enthaltend Buttersäure (44 mM) überführt und mit Ultraschall behandelt. Die Zelltrümmer wurden durch Zentrifugation entfernt. Der Überstand wurde für die Reinigung der Nitrilhydratase gemäss Tabelle 12 eingesetzt. Die Nitrilhydratase-Aktivität wurde gemäss Beispiel 1 bestimmt, statt der Zellsuspension wurden jedoch die jeweiligen Extrakte eingesetzt.

**Tabelle 12: Reinigung Nitrilhydratase aus Rhodococcus sp. FZ4.**

| **Reinigungsschritt** | **Proteingehalt [mg]** | **Gesamtaktivität [µmol/min]** | **Spezifische Aktivität [µmol/(min × mg)]** |
|---|---|---|---|
| Zellfreier Extrakt | 335 | 5881 | 17,6 |
| (NH₄)₂SO₄ Fällung | 198 | 6087 | 28,4 |
| DEAE-Sephacel | 73,0 | 3553 | 48,7 |
| Butyl-Toyopearl | 66,2 | 2035 | 30,7 |
| Phenyl-Sepharose | 26,2 | 890 | 34,0 |

### Beispiel 19

### Bestimmung des Molekulargewichtes der gereinigten Nitrilhydratase

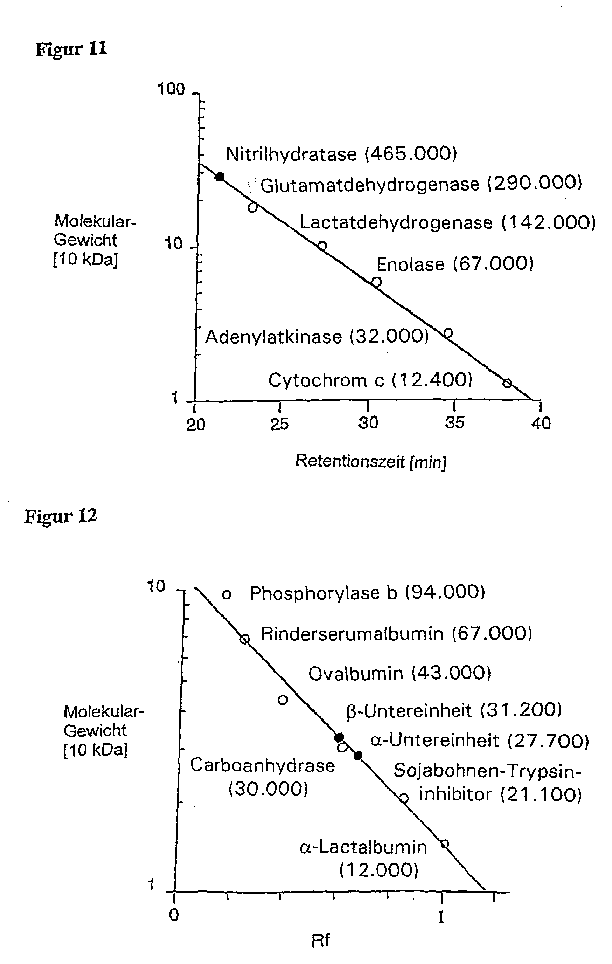

Das Molekulargewicht wurde mittels HPLC ermittelt (TSK gel G 300 SW (0,75 × 60 cm); Kaliumphosphatpuffer (0,1 M; pH 7,5) und Kaliumchlorid (0,2 M); 0,7 mL/min; 280 nm).
Das Molekulargewicht der Nitrilhydratase betrug 465 kDa (Fig. 11).
Die Nitrilhydratase besteht aus einer α-Untereinheit mit einem Molekulargewicht von 27,7 kDa und einer β-Untereinheit mit einem Molekulargewicht von 31,2 kDa (Fig. 12).

### Beispiel 20

### Thermische Stabilität der gereinigten Nitrilhydratase

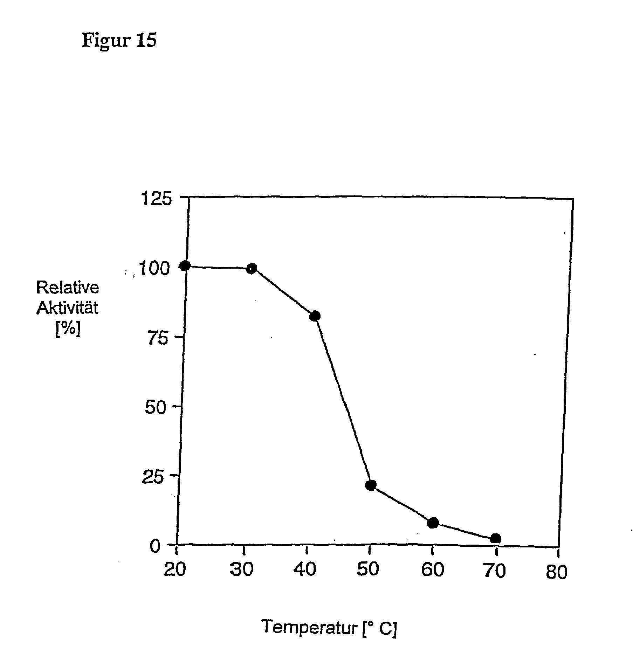

Eine Lösung bestehend aus Nitrilhydratase-Lösung (0,697 µmol/min; 0,025 mL) und Kaliumphosphatpuffer (0,1 M; pH 7,0; 0,475 mL) wurde 60 min bei unterschiedlichen Temperaturen im Bereich von 20 bis 70 °C inkubiert. Danach wurde die Lösung mit einem Eisbad auf 20 °C gekühlt und 3-Cyanpyridin (0,5 M; 0,500 mL) wurde hinzugegeben. Das Reaktionsgemisch wurde 10 min bei 20 °C inkubiert. Die Reaktion wurde durch Zugabe von MeOH gestoppt. Die Menge gebildetes Nikotinsäureamid wurde mittels HPLC gemäss Beispiel 1 bestimmt. Die Nitrilhydratase-Aktivität nahm nach 60 minütiger Inkubation bei Temperaturen von oberhalb 40 °C deutlich ab, nach 60 min Inkubation bei 50 °C betrug die Nitrilhydratase-Aktivität nur noch etwa 25% der ursprünglichen Aktivität (Fig. 15).

### Beispiel 21

### pH-Optimum der gereinigten Nitrilhydratase

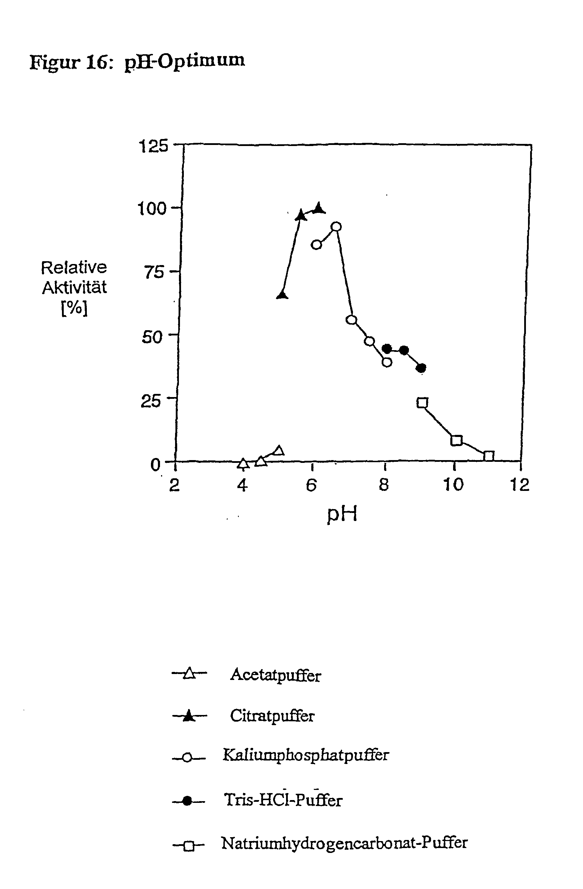

Ein Reaktionsgemisch bestehend aus 3-Cyanpyridin (0,5 M; 0,500 mL), Nitrilhydratase-Lösung (0,697 µmol/min; 0,025 mL) und unterschiedlichen Puffern im pH-Bereich von 4 bis 11 (0,1 M; 0,0475 mL) wurde 10 min bei 20 °C inkubiert. Die Reaktion wurde durch Zugabe von MeOH gestoppt. Die Menge gebildetes Nikotinsäureamid wurde mittels HPLC gemäss Beispiel 1 bestimmt. Das pH-Optimum der Nitrilhydratase lag im Bereich von 6,0 bis 6,5 (Fig. 16).

### Beispiel 22

### pH-Stabilität der gereinigten Nitrilhydratase

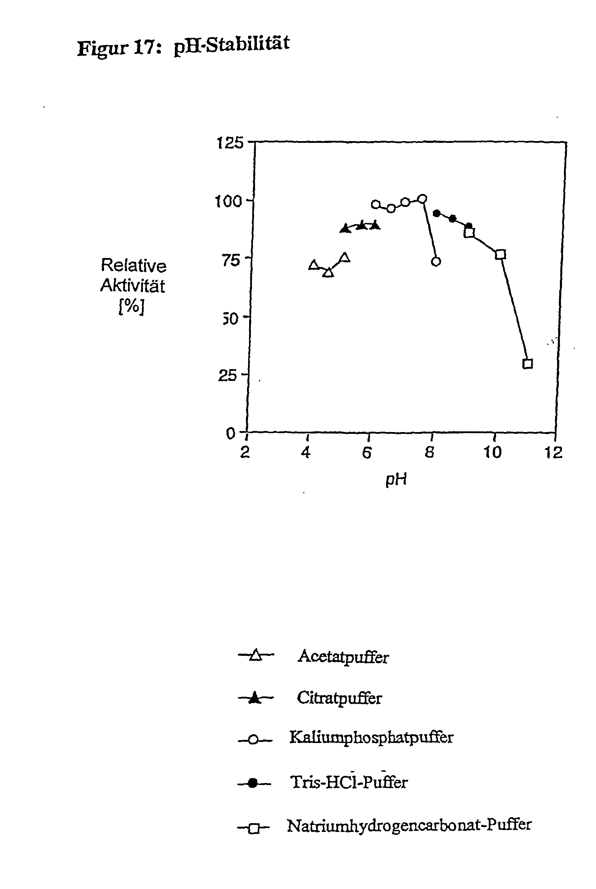

Eine Lösung bestehend aus Nitrilhydratase-Lösung (8,36 □mol/min; 0,47 mL), unterschiedlichen Puffern im pH-Bereich von 4,0 bis 11,0 (0,3 M; 0,10 mL) und destilliertem Wasser (0,03 mL) wurde 30 min bei 20 °C inkubiert. Ein Reaktionsgemisch bestehend aus einem Aliquot der inkubierten Nitrilhydratase-Lösung (0,05 mL), 3-Cyanpyridin (0,5 M; 0,5 mL) und dem jeweiligen Puffer (0,1 M; 0,45 mL) wurde 10 min bei 20 °C inkubiert. Die Realtion wurde durch Zugabe von MeOH gestoppt. Die Menge gebildetes Nikotinsäureamid wurde mittels HPLC gemäss Beispiel 1 bestimmt. Die Nitrilhydratase-Aktivität entsprach nach 60 minütiger Inkubation bei einem pH im Bereich von 6 bis 8 annähernd der ursprünglichen Nitrilhydratase-Aktivität (Fig. 17).

### Beispiel 23

### Substratspezifität der gereinigten Nitrilhydratase

Ein Reaktionsgemisch bestehend aus Nitrilhydratase-Lösung (0.695 µmol/min; 0,025 mL), unterschiedlichen Substraten (0,500 mL) und Kaliumphosphatpuffer (0,1 M; pH 7,0; 0.475 mL) wurde 5 bis 10 min bei 20 °C inkubiert. Die Konzentrationen der eingesetzten Substrate lagen im Bereich von 0,015 bis 0,250 M. Die Reaktion wurde durch Zugabe von MeOH gestoppt. Die Menge gebildetes Amid wurde mittels HPLC bestimmt. Die Ergebnisse sind in Tabelle 13 zusammengefasst. Die Nitrilhydratase weisst unter den getesteten Substraten bezüglich dem Substrat Acetonitril die höchste Aktivität auf.

**Tabelle 13: Substratspezifität der gereinigten Nitrilhydratase**

| **Substrat** | **Konzentration [M]** | **Relative Aktivität [%]** |
|---|---|---|
| Acetonitril | 0,2 | 1008 |
| Acrylnitril | 0,2 | 774 |
| Propionitril | 0,2 | 693 |
| Butyronitril | 0,2 | 578 |
| Crotonsäurenitril | 0,2 | 114 |
| 3-Cyanpyridin | 0,25 | 100^{a} |
| 4-Cyanpyridin | 0,125 | 92,8 |
| Benzonitril | 0,015 | 75,6 |
| m-Chlorbenzonitril | 0,015 | 66,5 |
| 2-Cyanpyridin | 0,125 | 36,7 |
| p-Chlorbenzonitril | 0,015 | 8,31 |
| Methacrylamid | 0,2 | 1,39 |
| o-Chlorbenzonitril | 0,015 | 0 |

| | | |
|---|---|---|
| ^{a} Gesamtaktivität: 4164 µmol/(min × mL). | | |

### Beispiel 24

### Einfluss von potentiellen Inhibitoren auf die gereinigte Nitrilhydratase

Eine Lösung bestehend aus Nitrilhydratase-Lösung (0,695 µmol/min; 0,025 mL), Kaliumphosphatpuffer (0,1 M; pH 7,0; 0,475 mL), destilliertem Wasser (0,150 mL) und unterschiedlichen potentiellen Inhibitoren (0,100 mL) wurde 5 min bei 20 °C inkubiert. Danach wurde 3-Cyanpyridin (1,0 M; 0,250 mL) hinzugegeben. Die Konzentration der Inhibitoren im Reaktionsgemisch betrug 1,0 mM. Das Reaktionsgemisch wurde 10 min bei 20 °C inkubiert. Die Reaktion wurde durch Zugabe von MeOH gestoppt. Die Menge gebildetes Nikotinsäureamid wurde mittels HPLC gemäss Beispiel 1 bestimmt. Die Ergebnisse sind in Tabelle 14 zusammengefasst. Unter den getesteten potentiellen Inhibitoren zeigen Hydroxylamin und Kaliumcyanid die stärkste Inhibitorwirkung.

**Tabelle 14: Einfluss von potentiellen Inhibitoren auf die gereinigte Nitrilhydratase**

| **Potentieller Inhibitor** | **Relative Aktivität [%]** |
|---|---|
| p-Chlormercuribenzoesäure^{a} | 187 |
| Tiron | 114 |
| Phenylmethansulfonylfluorid | 110 |
| 1,10-Phenanthrolin | 106 |
| Harnstoff | 106 |
| Dithiothreitol | 103 |
| EDTA^{b} | 100 |
| ----- | 100^{c} |
| Cysteamin | 99,1 |
| 8-Hydroxychinolin | 97,8 |
| 2,2'-Bipyridyl | 97,8 |
| Iodacetat | 96,7 |
| N-Ethylmaleinimid | 95,3 |
| Natriumazid | 93,5 |
| 5,5'-Dithiobis(2-nitrobenzoesäure)^{a} | 93,4 |
| Diethyldithiocarbamat | 93,3 |
| D-Cycloserin | 86,3 |
| Phenylhydrazin | 84,6 |
| 2-Mercaptoethanol | 76,3 |
| Hydroxylamin | 1,34 |
| Kaliumcyanid | 0 |

| | |
|---|---|
| ^{a} 0,1 mM. ^{b} Ethylendiamin-tetraessigsäure. ^{c} Gesamtaktivität: 3776 µmol/(min × mL) | |

### Beispiel 25

### Einfluss von Metallionen auf die Aktivität der gereinigte Nitrilhydratase

Der Einfluss von Metallionen auf die Aktivität der gereinigten Nitrilhydratase wurde gemäss Beispiel 24, jedoch unter Zusatz von Metallionen anstatt potentieller Inhibitoren durchgeführt. Die Konzentration der Metallionen im Reaktionsgemisch betrug 1,0 mM. Die Ergebnisse sind in Tabelle 15 zusammengefasst. Unter den getesteten Metallionen zeigen nur Silberkationen und zweiwertige Quecksilberkationen eine Inhibitorwirkung.

**Tabelle 15: Einfluss von Metallionen auf die Aktivität der gereinigte Nitrilhydratase**

| **Metallionen** | **Relative Aktivität [%]** |
|---|---|
| CuSO₄ | 177 |
| MnCl₂ | 123 |
| NiCl₂ | 123 |
| ZnSO₄ | 121 |
| FeSO₄ | 113 |
| CaCl₂ | 113 |
| CoCl₂ | 110 |
| FeCl₃ | 105 |
| ---- | 100^{a} |
| AgNO₃ | 0 |
| HgCl₂^{b} | 0 |

| | |
|---|---|
| ^{a} Gesamtaktivität: 3375 µmol/(min × mL). ^{b} 0,1 mM. | |

### Beispiel 26

### Bestimmung des K_{M}-Wertes für 3-Cyanpyridin bezüglich der gereinigten Nitrilhydratase

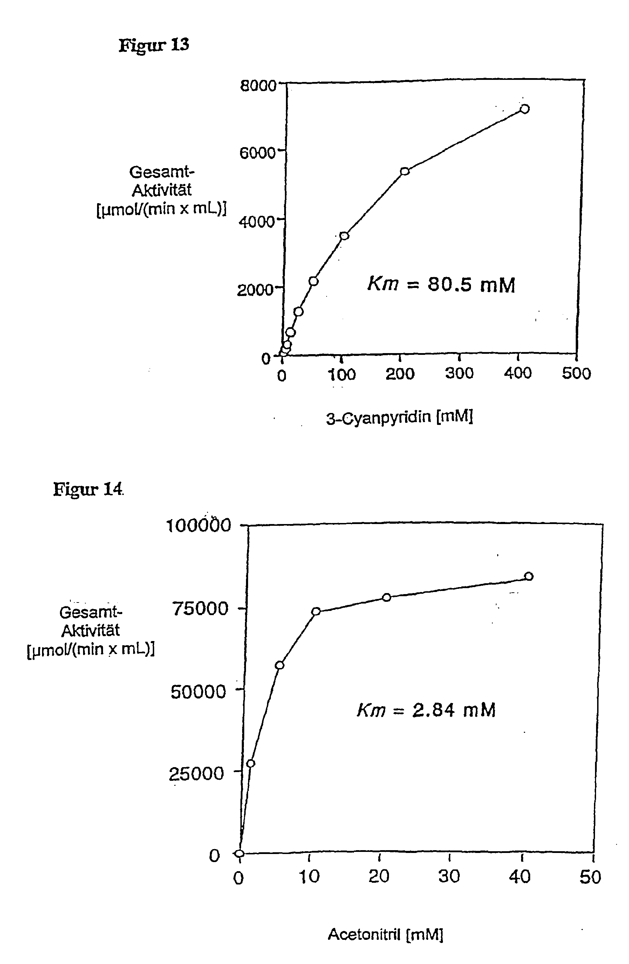

Zu einer Lösung bestehend aus Nitrilhydratase-Lösung (0,0697 µmol/min; 0,025 mL) und Kaliumphosphatpuffer (0,1 M; pH 7,0; 0,475 mL) wurde 3-Cyanpyridin in unterschiedlichen Konzentrationen (3,1-800 mM; 0,500 mL) hinzugegeben. Das Reaktionsgemisch wurde 10 min bei 20 °C inkubiert. Die Reaktion wurde durch Zugabe von Methanol gestoppt und die Menge gebildetes Nikotinsäureamid wurde mittels HPLC gemäss Beispiel 1 bestimmt.
Der K_{M}-Wert für 3-Cyanpyridin betrug 80,5 mM (Fig 13).

### Beispiel 27

### Bestimmung des K_{M}-Wertes für Acetonitril bezüglich der gereinigten Nitrilhydratase

Zu einer Lösung bestehend aus Nitrilhydratase-Lösung (0,0697 µmol/min; 0,025 mL) und Kaliumphosphatpuffer (0,1 M; pH 7,0; 0,475 mL) wurde Acetonitril in unterschiedlichen Konzentrationen (2,5-80 mM; 0,500 mL) hinzugegeben. Das Reaktionsgemisch wurde 10 min bei 20 °C inkubiert. Die Reaktion wurde durch Zugabe von Methanol gestoppt und die Menge gebildetes Acetamid wurde mittels HPLC gemäss Beispiel 14 bestimmt. Der K_{M}-Wert für Acetonitril betrug 2,84 mM (Fig 14).

## Patentansprüche

1. Mikroorganismen der Spezies *Rhodococcus sp.* FZ4, wie hinterlegt unter der DSM Nummer 13597.

2. Mikroorganismen nach Anspruch 1, **dadurch gekennzeichnet, dass** sie befähigt sind, eine Konzentration an Acetonitril von mindestens 3 M zu tolerieren.

3. Mikroorganismen nach Anspruch 2, **dadurch gekennzeichnet, dass** sie befähigt sind, 3-Cyanpyridin zu Nikotinsäureamid umzusetzen.

4. Mikroorganismen nach mindestens einem der Ansprüche 1 bis 3, **dadurch gekennzeichnet, dass** Mycolsäuren mit einer Kettenlänge von C₄₈ vorhanden sind.

5. Enzymextrakte, erhältlich aus den Mikroorganismen nach mindestens einem der Ansprüche 1 bis 4.

6. Nitrilhydratase, erhältlich aus den Mikroorganismen nach mindestens einem der Ansprüche 1 bis 4.

7. Nitrilhydratase, **gekennzeichnet durch**
a) einen K_{M}-Wert für das Substrat Acetonitril von 2,84 ± 1,00 mM und für das Substrat 3-Cyanpyridin von 80,5 ± 15,0 mM
b) ein pH-Optimum von pH 6,5 ± 1,0
c) ein Molekulargewicht von 465 ±50 kDa, umfassend α- und β-Untereinheiten, bestimmt durch HPLC.

8. Verfahren zur Herstelung von Amiden der allgemeinen Formel
R¹-CONH₂ III
worin R¹ einen C₁₋₆-Alkylrest, eine C₂-₆-Alkenylgruppe oder eine Gruppe der allgemeinen Formel 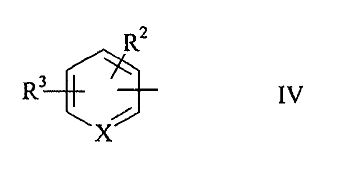 bedeutet, worin X ein Stickstoffatom oder -CH= bedeutet und R² und R³ unabhängig voneinander ein Wasserstoffatom, ein Halogenatom, eine C₁₋₆-Alkylgruppe oder eine C₂₋₆-Alkenylgruppe bedeuten, **dadurch gekennzeichnet, dass** ein Nitril der allgemeinen Formel
R¹-CN II
worin R¹ die genannte Bedeutung hat, mittels eines Mikroorganismus nach mindestens einem der Ansprüchen 1 bis 4, eines Enzymextrakts nach Anspruch 5 oder mittels eines Enzyms nach mindestens einem der Ansprüche 6 oder 7 umgesetzt wird.

9. Verfahren nach Anspruch 8, **dadurch gekennzeichnet, dass** als Nitril 3-Cyanpyridin oder Acetonitril eingesetzt wird.

10. Verfahren nach mindestens einem der Ansprüche 8 oder 9, **dadurch gekennzeichnet, dass** die Umsetzung bei einer Temperatur von 5 bis 50°C und bei einem pH von 5 bis 10 durchgeführt wird.

11. Verwendung der Mikroorganismen nach mindestens einem der Ansprüche 1 bis 4 zur Beseitigung von Acetonitril-Abfällen.

## Claims

1. Microorganisms of the species *Rhodococcus sp.* FZ4, as deposited under the DSM number 13597.

2. Microorganisms according to Claim 1, **characterized in that** they are capable of tolerating an acetonitrile concentration of at least 3 M.

3. Microorganisms according to Claim 2, **characterized in that** they are capable of reacting 3-cyanopyridine to give nicotinamide.

4. Microorganisms according to at least one of Claims 1 to 3, **characterized in that** mycolic acids having a chain length of C₄₈ are present.

5. Enzyme extracts, obtainable from the microorganisms according to at least one of Claims 1 to 4.

6. Nitrile hydratase, obtainable from the microorganisms as claimed in at least one of Claims 1 to 4.

7. Nitrile hydratase, **characterized by**
a) a K_{M} value of 2.84 ± 1.00 mM for the substrate acetonitrile and of 80.5 ± 15.0 mM for the substrate 3-cyanopyridine
b) a pH optimum of pH 6.5 ± 1.0
c) a molecular weight of 465 ± 50 kDa, comprising α- and β-subunits, determined by HPLC.

8. Method for preparing amides of the general formula
R¹-CONH₂ III
in which R¹ is a C₁₋₆-alkyl group, a C₂₋₆-alkenyl group or a group of the general formula 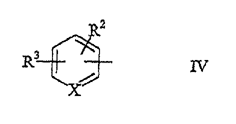 in which X is a nitrogen atom or -CH=, and R² and R³ are independently of one another a hydrogen atom, a halogen atom, a C₁₋₆-alkyl group or a C₂₋₆-alkenyl group, **characterized in that** a nitrile of the general formula
R¹-CN II
in which R¹ is defined as above, is converted by means of a microorganism according to at least one of Claims 1 to 4, an enzyme extract according to Claim 5 or by means of an enzyme according to at least one of Claims 6 and 7.

9. Method according to Claim 8, **characterized in that** the nitrile used is 3-cyanopyridine or acetonitrile.

10. Method according to at least one of Claims 8 and 9, **characterized in that** the reaction is carried out at a temperature of from 5 to 50°C and at a pH of from 5 to 10.

11. Use of the microorganisms according to at least one of Claims 1 to 4 for removing acetonitrile waste.

## Revendications

1. Micro-organismes de l'espèce *Rhodococcus sp.* FZ4, comme déposé sous le DSM N° 13597.

2. Micro-organismes selon la revendication 1,
**caractérisés en ce qu'**ils sont capables de tolérer une concentration en acétonitrile d'au moins 3 M.

3. Micro-organismes selon la revendication 2,
**caractérisés en ce qu'**ils sont capables de transformer la 3-cyanopyridine en nicotinamide.

4. Micro-organismes selon au moins l'une des revendications 1 à 3, **caractérisés en ce que** des acides mycoliques présentant une longueur de chaîne C₄₈ sont présents.

5. Extraits enzymatiques pouvant être obtenus à partir des micro-organismes selon au moins l'une des revendications 1 à 4.

6. Nitrile hydratase pouvant être obtenue à partir des micro-organismes selon au moins l'une des revendications 1 à 4.

7. Nitrile hydratase, **caractérisée par**
a) une valeur de K_{M} pour le substrat acétonitrile de 2,84 ± 1,00 mM et pour le substrat 3-cyanopyridine de 80,5 ± 15,0 mM,
b) un optimum de pH de 6,5 ± 1,0,
c) un poids moléculaire de 465 ± 50 kDa, comprenant des sous-motifs α et β, déterminé par HPLC.

8. Procédé de préparation d'amides de formule générale
R¹-CONH₂ III
dans laquelle R¹ représente un groupe alkyle en C₁₋₆, un groupe alcényle en C₂₋₆ ou un groupe de formule générale 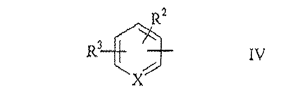 dans laquelle X représente un atome d'azote ou un groupe -CH=, et R² et R³ représentent, indépendamment l'un de l'autre, un atome d'hydrogène, un atome d'halogène, un groupe alkyle en C₁₋₆ ou un groupe alcényle en C₂₋₆, **caractérisé en ce qu'**un nitrile de formule générale
R¹-CN II
dans laquelle R¹ présente la signification mentionnée, est transformé au moyen d'un micro-organisme selon au moins l'une des revendications 1 à 4, d'un extrait enzymatique selon la revendication 5 ou au moyen d'un enzyme selon au moins l'une des revendications 6 ou 7.

9. Procédé selon la revendication 8, **caractérisé en ce que** l'on utilise la 3-cyanopyridine ou l'acétonitrile en tant que nitrile.

10. Procédé selon au moins l'une des revendications 8 ou 9, **caractérisé en ce que** la réaction est effectuée à une température de 5 à 50°C et à un pH de 5 à 10.

11. Utilisation des micro-organismes selon au moins l'une des revendications 1 à 4 pour l'élimination de résidus d'acétonitrile.
